(19) Europäisches Patentamt
European Patent Office
Office européen des brevets

(11) EP 1 464 649 B1

(12) **EUROPEAN PATENT SPECIFICATION**

(45) Date of publication and mention of the grant of the patent:
**20.10.2010 Bulletin 2010/42**

(21) Application number: **03398005.3**

(22) Date of filing: **21.08.2003**

(51) Int Cl.:
*C07H 7/06* (2006.01) *C07H 9/04* (2006.01)
*C07D 309/32* (2006.01) *C07H 3/10* (2006.01)
*A01N 43/08* (2006.01) *A01N 43/16* (2006.01)
*A01N 43/90* (2006.01)

(54) **Sugar derivatives comprising oxiranes or alpha,beta-unsaturated gamma-lactones, process for their preparation and their utilisation as pesticides**

Zuckerderivate bestehend aus Oxirane oder alpha,beta-ungesättigte gamma-Laktone, Verfahren zu ihrer Herstellung und ihre Bentzung als Pestizide

Dérivés de sucre comprenant des oxiranes ou des gamma-lactones, alpha,beta-insaturées, procédé de fabrication et utilisation comme pesticides

(84) Designated Contracting States:
**AT BE BG CH CY CZ DE DK EE ES FI FR GB GR HU IE IT LI LU MC NL PT RO SE SI SK TR**

(30) Priority: **03.04.2003 PT 10293503**

(43) Date of publication of application:
**06.10.2004 Bulletin 2004/41**

(60) Divisional application:
**09002251.8 / 2 172 473**

(73) Proprietors:
- **Instituto Politécnico de Santarém / Escola Superior Agraria**
  **2001-904 Satarém (PT)**
- **Faculdade de Ciencias da Universidade de Lisboa**
  **1749-016 Lisboa (PT)**
- **The University of Newcastle**
  **Jesmond,**
  **Newcastle Upon Tyne NE1 7RU (GB)**

(72) Inventors:
- **Guerra Justino, Jorge Alberto**
  **2000-019 Santarém (PT)**
- **Pilar grases santos Silva Reuter, Amélia**
  **1000-239 Lisboa (PT)**
- **Lukeba Canda, Tana**
  **Terra Nova**
  **Municipio do Rangel**
  **Luanda (AO)**
- **Wilkins, Richard**
  **Newcastle-upon-Tyne NE2 2DR (GB)**

(74) Representative: **Pereira da Cruz, Joao**
**J. Pereira da Cruz, S.A.**
**Rua Vitor Cordon, 14**
**1249-103 Lisboa (PT)**

(56) References cited:
**US-A- 4 994 572**

- **RAUTER A P ET AL: "Efficient synthesis of alpha, beta-unsaturated gamma-lactones linked to sugars" TETRAHEDRON: ASYMMETRY, ELSEVIER SCIENCE PUBLISHERS, AMSTERDAM, NL, vol. 12, no. 8, 21 May 2001 (2001-05-21), pages 1131-1146, XP004245861 ISSN: 0957-4166**
- **A.P. RAUTER ET AL.: "Synthetic, fungicidal unsaturated gamma-lactones attached to furanosidic systems. Configurational determination by nuclear Overhauser effect" J. CARBOHYDRATE CHEM., vol. 14, 1995, pages 929-948, XP008033216**
- **S. HANESSIAN ET AL.: "Mild and efficient preparation of gamma-substituted alfa, beta-unsaturated gamma-butyrolactones from epoxides" J. CHEM. SOC., CHEM. COMMUN., 1986, pages 754-755, XP002289672**
- **PANFIL I ET AL: "1,3-DIPOLAR CYCLOADDITION OF NITRONES TO SUGAR ENLACTONES" TETRAHEDRON, ELSEVIER SCIENCE PUBLISHERS, AMSTERDAM, NL, vol. 47, no. 48, 1991, pages 10087-10094, XP002041211 ISSN: 0040-4020**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **J. LEE ET AL.: "Conformationally constrained analogues of diacylglycerol. 13. Protein kinase C ligands based on templates derived from 2,3-dideoxy-L-erythro(threo)-hexono-1,4-la ctone and 2-deoxyapiolactone" J. MED. CHEM., vol. 39, 1996, pages 4912-4919, XP002419334**
- **R.D. EVANS, J.H. SCHAUBLE: "Synthesis of alfa-iodocarbonyl compounds using bis(sym-collidine)iodine(I) tetrafluoroborate/dimethyl sulfoxide" SYNTHESIS, 1986, pages 727-730, XP002419335**
- **A. VARGAS-BERENGUEL ET AL.: "Synthesis of 6-deoxyheptose derivatives via cyclic sulfates and oxetanes" SYNTHESIS, 1999, pages 1778-1786, XP002419336**

## Description

### Field of the Invention

The present invention relates to the use of sugar derivatives comprising or α,β-unsaturated or α-substituted γ-lactones as insecticides, which are particularly effective against fruit fly, domestic fly and white fly.

### Background of the Invention

Compounds with the α,β-unsaturated γ-lactone moiety in their structure occur in the plant kingdom, as metabolites of lichens and fungi,[1] as sesquiterpene derivatives[2] or as steroid glycosides.[3]

Natural products possessing this structural element are also components of animal species such as sponges.[4]

Many of these compounds exhibit a variety of biologic activities such as antifungal, insecticidal, antibacterial, phytotoxic and anti-inflammatory. Some of them are antibiotics, potential anticancer agents and cyclooxygenase or phospholipase $A_2$ inhibitors.[5]

Due to their biological importance, several synthetic methods have been developed for the preparation of α, β-unsaturated γ-lactones. The synthesis of the endocyclic lactones (α,β-butenolides) is reported in the literature, and includes mercuration-carbonylation of propargylic alcohols,[6] condensation of 2,5-bis(trimethylsiloxy)furans with carbonyl compounds in the presence of titanium tetrachloride[7] and various transformations of $C_3$ synthons, such as, for example, glycidaldehyde.[8]

Other references report methods for the synthesis of γ-alkylidene-α,β-butenolides[9] and for the preparation of α,β-butenolide derivatives with insect antifeedant activity.[10]

A method for the preparation of the exocyclic type lactones involves the reaction of 2-(bromomethyl)acrylic acid in the presence of indium with carbonyl compounds, to give α-methylene-γ-butyrolactones in 7-96% yield.[11]

Previous work reports the synthesis of butenolides through the condensation of sugar epoxides with the dianion of phenylselenoacetic acid, followed by hydrolysis and subsequent oxidation of the intermediate phenylselenolactone.[12,13,14] The nucleophilic opening of the oxirane is stereospecific, the configuration of the stereogenic centre in the final lactone being determined by the centre of chirality of the starting epoxide.

Another method for the synthesis of α,β-unsaturated γ-lactones involves a Reformatsky type reaction of a ketosugar or a dialdofuranose with ethyl bromomethylacrylate and zinc in THF under reflux.[13,14,15]

Ethyl bromomethylacrylate and zinc-silver/graphite at -78°C have been successfully applied to the synthesis of hydroxyesters from cyclic ketones,[16] ketosugars and a 2,3-*O*-isopropylidene-D-erythronolactone[17] and to the synthesis of α,β-unsaturated γ-lactones from some ketosugars.[16]

The synthesis of 3-ulosonic acids via a samarium iodide Reformatsky reaction of aldonolactones was also reported.[18]

Some of this type of compounds, reported in the literature, have fungicidal efficacy.[13]

Epoxy sugars are versatile intermediates in organic synthesis, due to the ease of their preparation from a variety of starting materials and due to their susceptibility to reactions for example with electrophiles, nucleophiles, acids and bases. Furthermore, epoxides are part of a range of compounds recognised as active principles, with biological and pharmacological activity.[19] Reference can be made for example to cytotoxic metabolites, namely crotepoxide, pipoxide and senepoxide, the latter playing an important role in plants as an antiparasitic agent.[20]

Methods for the preparation of epoxysugars use halohydrins as intermediate compounds,[21] and also amino-sugars,[22] tosylates and/or mesylates,[23] vicinal diols,[24,25] glycals[26] and carbonyl compounds.[27]

### Summary of the Invention

This invention is related to the the use, as insecticides, of compounds of general formula (I) described further on.

These compounds possess efficacy as insecticides with high toxicity to fruit fly (*Drosophila melanogaster*), house fly (*Musca domestica*) and white fly *(Trialeurodes vaporarium).*

On the other hand the compounds are not toxic to brine shrimps (*Artemia salina*), the reference organisms in assays to evaluate the potential toxicity hazard to organisms in ecosystems.

### Detailed Description of the Invention

The first object of the invention is the use, as insecticides, of a family of compounds of general formula (I):

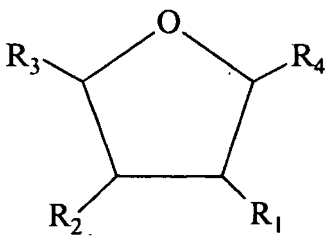

(I)

wherein

$R_1$ and $R_2$ represent, independently, hydrogen, halogen, alkoxy, substituted alkoxy or an ester group; or

$R_1$ and $R_2$, together with the carbon atoms to which they are attached, represent an oxirane ring; or

$R_1$ and $R_2$, taken together, represent an akylidenedioxy or substituted alkylidenedioxy group; and

$R_3$ represents a group of formula

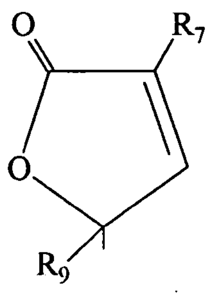

or

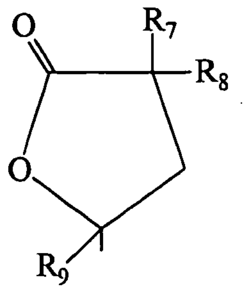

wherein
$R_7$ represents hydrogen or alkyl,
$R_8$ represents phenylsulfanyl, phenylselenyl, phenylsulfoxy or phenylselenoxy, and
$R_9$ represents hydrogen, ethoxycarbonyl or carbamoyl; and

$R_4$ represents, independently, hydrogen, alkoxy or substituted alkoxy, or

$R_1$ and $R_4$, taken together, represent an alkylidenedioxy or substituted alkylidenedioxy group.

Preferably, these compounds are used with particular efficacy as insecticides of high toxicity for controlling fruit fly (*Drosophila melanogaster*), house fly (*Musca domestica*) and white fly (*Trialeurodes vaporarium*).

In a first embodiment, the invention relates to the use, as insecticides, of a subgroup of compounds within the family of compounds of formula (I), wherein

$R_1$ and $R_2$ represent, independently, hydrogen, alkoxy, substituted alkoxy or an ester group, or

$R_1$ and $R_2$, together with the carbon atoms to which they are attached, represent an oxirane ring; or

$R_1$ and $R_2$, taken together, represent an alkylidenedioxy or substituted alkylidenedioxy group; and

$R_3$ represents a group of formula

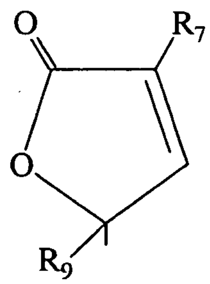

wherein
$R_7$ represents hydrogen or alkyl, and
$R_9$ represents hydrogen, ethoxycarbonyl or carbamoyl; and

$R_4$ represents, independently, hydrogen, alkoxy or substituted alkoxy, or

$R_1$ and $R_4$, taken together, represent an alkylidenedioxy or a substituted alkylidenedioxy group.

Preferred within this subgroup are the compounds of general formula (IA):

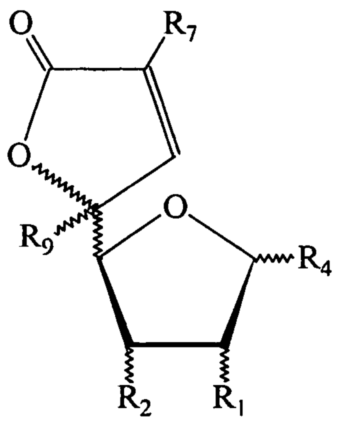

(IA)

wherein $R_1$, $R_2$, $R_4$, $R_7$ and $R_9$ have the meanings indicated for this first embodiment.

Especially preferred are the compounds of formula (IA), wherein $R_1$ and $R_4$, taken together, represent the isopropylidenedioxy group, $R_2$ represents benzyloxy, $R_7$ represents methyl and $R_9$ represents hydrogen.

Among these, the most especially preferred are the D-*gluco* derivative, *i.e.* 3-*O*-benzyl-6,7-dideoxy-1,2-*O*-isopropylidene-7-methyl-α-D-*gluco*-oct-6-enefuranurono-8,5-lactone or the D-*alo* derivative, *i.e.* 3-*O*-benzyl-6,7-dideoxy-1,2-*O*-isopropylidene-7-methyl-α-D-*alo*-oct-6-enefuranurono-8,5-lactone.

Also particularly preferred are compounds of formula (IA), wherein $R_1$ and $R_4$, taken together, represent the isopropylidenedioxy group, $R_2$ represents benzyloxy and $R_7$ and $R_9$ represent hydrogen.

Among these, the most especially preferred are the D-*alo* derivative, *i.e.* 3-*O*-benzyl-6,7-dideoxy-1,2-*O*-isopropylidene-α-D-*alo*-oct-6-enefuranurono-8,5-lactone and the D-*gluco* derivative, *i.e.* 3-*O*-benzyl-6,7-dideoxy-1,2-*O*-isopropylidene-α-D-*gluco*-oct-6-enefuranurono-8,5-lactone.

In a second embodiment, the invention relates to the use, as insecticides, of a subgroup of compounds within the family of compounds of formula (I), wherein

$R_1$ and $R_2$ represent, independently, hydrogen, alkoxy or substituted alkoxy, or

$R_1$ and $R_2$, together with the carbon atoms to which they are attached, represent an oxirane ring; or

$R_1$ and $R_2$, taken together, represent an alkylidenedioxy or a substituted alkylidenedioxy group; and

$R_3$ represents a group of formula

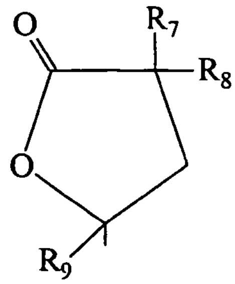

wherein
$R_7$ represents hydrogen or alkyl,
$R_8$ represents phenylsulfanyl, phenylselenyl, phenylsulfoxy or phenylselenoxy, and
$R_9$ represents hydrogen, ethoxycarbonyl or carbamoyl; and

$R_4$ represents, independently, hydrogen, alkoxy or substituted alkoxy, or

$R_1$ and $R_4$, taken together, represent an alkylidenedioxy or substituted alkylidenedioxy group.

Preferred within this subgroup are the compounds of general formula (IC):

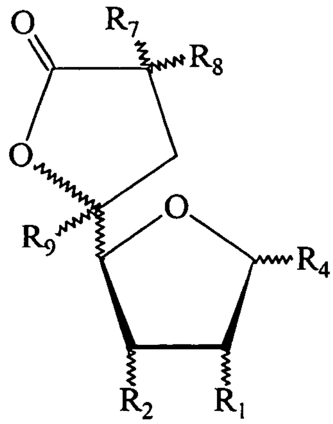

(IC)

wherein $R_1$, $R_2$, $R_4$, $R_7$, $R_8$ and $R_9$ have the meanings indicated for this second embodiment.

Especially preferred are the compounds of formula (IC), wherein $R_1$ and $R_2$, together with the carbon atoms to which they are attached, form an oxirane ring, $R_4$ represents methoxy, $R_7$ represents methyl, $R_8$ represents phenylselenyl and $R_9$ represents hydrogen.

Among these, the most especially preferred is the 2,3-anhydro-β-L-*gulo* derivative, *i.e.* methyl (7*R*)- and methyl (7*S*)-2,3-anhydro-6,7-dideoxy-7-methyl-7-phenylselenyl-β-L-*gulo*-octofuranurono-8,5-lactone.

Also especially preferred are compounds of formula (IC), wherein $R_1$ and $R_4$, taken together, form an isopropylidenedioxy group, $R_2$ represents hydrogen, $R_7$ represents methyl, $R_8$ represents phenylselenyl and $R_9$ represents hydrogen.

Among these, the most especially preferred is the D-*ribo* derivative, *i.e.* (7*R*)- and (7*S*)-3,6,7-trideoxy-1,2-*O*-isopropylidene-7-methyl-7-phenylselenyl-α-D-*ribo*-octofuranurono-8,5-lactone.

Especially preferred are also the compounds of formula (IC), wherein $R_1$ and $R_4$, taken together, form an isopropylidenedioxy group, $R_2$ represents benzyloxy, $R_7$ represents methyl, $R_8$ represents phenylselenyl and $R_9$ represents hydrogen.

Among these, the most especially preferred is the D-*gluco* derivative, *i.e.* (7*R*)- and (7*S*)-3-*O*-benzyl-6,7-dideoxy-1,2-*O*-isopropylidene-7-methyl-7-phenylselenyl-α-D-*gluco*-octofuranurono-8,5-lactone.

The compounds employed in the present invention may be obtained by the preparation processes described hereafter.

**Synthesis of compounds of Formula (IA)**

Compounds of formula (IA) can be prepared by oxidation of phenylsulfanyllactones with *m*-chloroperbenzoic acid or with sodium metaperiodate, leading to the formation of sulfoxides which, upon pyrolysis in toluene at reflux, provide the corresponding butenolides. The transformation of the phenylselenyllactones into butenolides results from the oxidation with hydrogen peroxide, under acid catalysis. This synthesis is summarized in the following **Scheme 1.**

**Scheme 1**

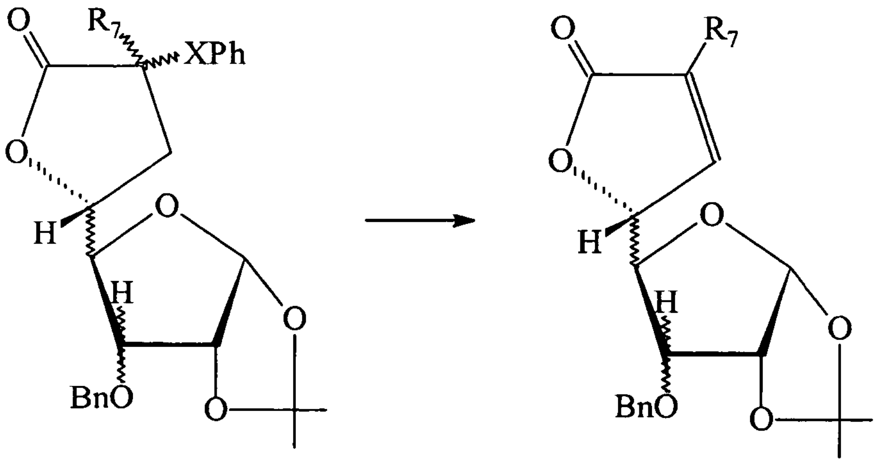

(IC')

(IA')

In **Scheme 1**, a lactone of formula (IC') [compound of formula (IC), wherein $R_1$ and $R_4$ represent, taken together, isopropylidenedioxy, $R_2$ represents benzyloxy, $R_7$ represents hydrogen or methyl, $R_8$ represents XPh, wherein X represents S or Se, and $R_9$ represents hydrogen] is converted into a butenolide of formula (IA') [compound of formula (IA), wherein $R_1$ and $R_4$ represent, taken together, isopropylidenedioxy, $R_2$ represents benzyloxy, $R_7$ represents hydrogen or methyl and $R_9$ represents hydrogen]. This conversion is carried out in the presence of *m*-chloroperbenzoic acid (when X represents S) and toluene at reflux, or in the presence of hydrogen peroxide, in acid medium (when X represents Se), at temperatures between -20°C and room temperature.

**Synthesis of compounds of Formula (IC)**

Compounds of formula (IC) can be prepared by reaction of the corresponding epoxide precursor with dianions, namely: dianion of phenylselenoacetic acid, phenylselenopropionic acid and phenylthioacetic acid, which upon cyclization in acid medium yield the corresponding phenylselenyllactones or phenylsulfanyllactones, according to **Schemes 3a** and 3b.

**Scheme 3a**

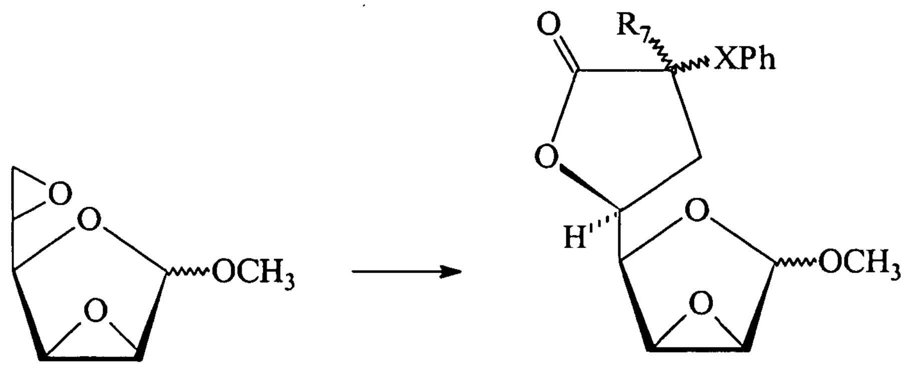

(ID') (IC")

In **Scheme 3a**, a diepoxide of formula (ID') [compound of formula (ID), wherein $R_1$ and $R_2$ represent, taken together, oxiranyl and $R_4$ represents methoxy] is converted into a lactone of formula (IC") [compound of formula (IC), wherein $R_1$ and $R_2$ represent, taken together, oxiranyl, $R_4$ represents methoxy, $R_7$ represents hydrogen or methyl, $R_8$ represents XPh, wherein X represents S or Se, and $R_9$ represents hydrogen]. This conversion is carried out by treatment of (ID') with lithium diisopropropylamide in tetrahydrofuran, at a temperature between -10°C and 10°C, followed by reaction with a compound of formula $PhXCHR_7CO_2H$ (wherein X represents S or Se e $R_7$ represents hydrogen or methyl).

**Scheme 3b**

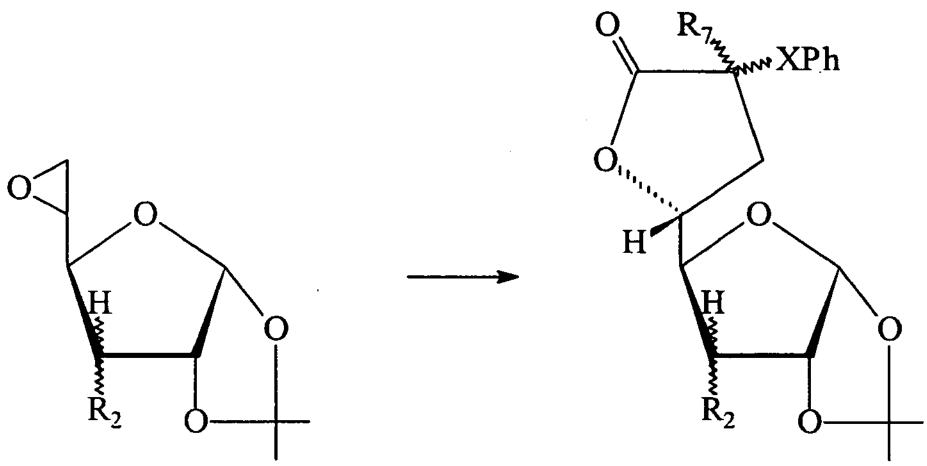

(IE') (IC"')

In **Scheme 3b**, following a procedure similar to that of **Scheme 3a,** an epoxide of formula (IE') [compound of formula (IE), wherein $R_1$ and $R_4$ represent, taken together, isopropylidenedioxy and $R_2$ represents benzyloxy or hydrogen] is converted into a lactone of formula (IC"') [compound of formula (IC), wherein $R_1$ and $R_4$ represent, taken together, isopropylidenedioxy, $R_2$ represents benzyloxy or hydrogen, $R_7$ represents hydrogen or methyl, $R_8$ represents XPh, wherein X represents S or Se, and $R_9$ represents hydrogen].

A second object of the invention is a method for controlling insects, particularly fruit fly, house fly and white fly. Said method comprises the application of an effective amount of compounds of formula (I) to said insects or their locus.

## EXPERIMENTAL

### Preparation Examples

Example 1

Preparation of 3-*O*-benzyl-6,7-dideoxy-1,2-*O*-isopropylidene-7-methyl-α-D-*gluco*-oct-6-enefuranurono-8,5-lactone **(1)**

Glacial acetic acid (1 drop) was added to a solution of (7*R*)- and (7*S*)-3-*O*-benzyl-6,7-dideoxy-1,2-*O*-isopropylidene-7-methyl-7-phenylselenyl-α-D-*gluco*-octofuranurono-8,5-lactone (130 mg, 0.26 mmol) in anhydrous tetrahydrofuran (0.26 mL) at 0°C. 30% $H_2O_2$ (6.70 eq.) was added dropwise and the reaction mixture was stirred for 45 min at 0 °C. Extraction with a saturated solution of $NaHCO_3$ was followed by extraction with $CH_2Cl_2$ (3x2 mL). The organic phase was dried over sodium sulphate and evaporated under vacuum. The residue was purified by low pressure column chromatography to provide the title compound (45 mg, 64%) and unreacted starting material (26 mg, 20%); $R_f$: 0,40 (ethyl acetate/*n*-hexane 1:4); $[\alpha]_D^{20}$ (c 1.0; $CHCl_3$); IR (neat): 1770 (C=O), 1378 (C-O, isopropyl), 1662 (C=C) $cm^{-1}$; $^1H$ NMR (300 MHz, $CDCl_3$): δ 7.35-7.30 (m, 6H, H-6, Ph), 5.93 (d, 1H, H-1, $J_{1,2}$ = 3.6 Hz), 5.17 (dd, 1H, H-5, $J_{4,5}$ = 8.6 Hz, $J_{5,6}$ = 1.5 Hz), 4.69 (s, 2H, $OCH_2Ph$), 4.63 (d, 1H, H-2), 4.14 (d, 1H, H-3, $J_{3,4}$ = 3.0 Hz), 3.91 (dd, 1H, H-4), 1.93 (s, 3H, Me-7), 1.48 (s, 3H, Me, isopropyl), 1,25 (s, 3H, Me, isopropyl); $^{13}C$ NMR (75.43 MHz, $CDCl_3$): δ 174.0 (C=O), 148.7 (C-6), 137.1 (Cq, Ph), 130.2 (C-7), 128.5; 128.1; 127.8 (Ph), 112.3 (Cq, isopropyl), 105.3 (C-1), 82.3 (C-2), 81.8 (C-3), 81.5 (C-4), 76.8 (C-5), 72.8 ($OCH_2Ph$), 10.7 (Me-7), 26.73 (Me, isopropyl), 26.11 (Me, isopropyl). Anal. calcd for $C_{19}H_{22}O_6$ (346.35): C 65.89; H 6.39; Found: C 65.57; H 6.45%.

Example 2

Preparation of 3-*O*-benzyl-6,7-dideoxy-1,2-*O*-isopropylidene-7-methyl-α-D-*alo*-oct-6-enefuranurono-8,5-lactone **(2)**

Following a procedure similar to that of Example 1, starting from (7*R*)- and (7*S*)-3-*O*-benzyl-6,7-dideoxy-1,2-*O*-isopropylidene-7-methyl-7-phenylselenyl-α-D-*gluco*-octofuranurono-8,5-lactone[15] (100 mg, 0.2 mmol), the title compound was obtained (40 mg, 73% taking into account the starting material that reacted), with recovery of unreacted starting material (20 mg, 20%); $R_f$: 0.23 (ethyl acetate/n-hexane 1:3); $[\alpha]_D^{20}$ (c 1.0; $CHCl_3$); IR (neat): 1786 (C=O), 1384 (C-O, isopropyl), 1662 (C=C) $cm^{-1}$; $^1H$ NMR (300 MHz, $CDCl_3$): δ 7.41-7.30 (m, 5H, Ph), 7.25 (s, 1H, H-6), 5.77 (d, 1H, H-1, $J_{1,2}$ = 3.6 Hz), 5.16 (br s, 1H, H-5), 4.68; 4.64 (part A of AB system, $OCH_2Ph$, $J_{AB}$ =12 Hz), 4.56 (t, 1H, H-2, $J_{2,3}$ = 4.4 Hz), 4.49; 4.45 (part B of AB system, 4.32 (dd, 1H, H-4, $J_{3,4}$ = 8.7 Hz, $J_{4,5}$ = 3.0 Hz), 3.65 (dd, 1H, H-3), 1.93 (s, 3H, Me-7), 1.55 (s, 3H, Me, isopropyl), 1.39 (s, 3H, Me, isopropyl); $^{13}C$ NMR (75.43 MHz, $CDCl_3$): δ 173.8 (C=O), 145.3 (C-6), 136.9 (Cq, Ph), 130.4 (C-7), 128.5; 128.2; 128.0 (Ph), 113.3 (Cq, isopropyl), 104.1 (C-1), 79.3 (C-5), 78.6 (C-4), 72.1 ($OCH_2Ph$), 76.6 (C-2), 75.5 (C-3), 26.8 (Me, isopropyl), 26,5 (Me, isopropyl), 10.7 (Me-7). Anal. calcd for $C_{19}H_{22}O_6$ (346.35): C 65.89; H 6.39; Found: C 65.63; H 6.48%.

Example 3

Preparation of 3-*O*-benzyl-6,7-dideoxy-1,2-*O*-isopropylidene-α-D-*alo*-oct-6-enefuranurono-8,5-lactone **(3)**

Following a procedure similar to that of Example 1, starting from (7*R*)- and (7*S*)-3-*O*-benzyl-6,7-dideoxy-1,2-*O*-isopropylidene-7-phenylselenyl-α-D-*alo*-octofuranurono-8,5-lactone[15] (130 mg, 0.3 mmol) the title compound was obtained (100 mg, 91 %). $R_f$ 0,60 (ethyl acetate/*n*-hexane 1:1); $[\alpha]_D^{20}$ (c 0,5; $CH_2Cl_2$); IR (KBr): 1774 (C=O), 1382 (C-O, isopropyl), 1662 (C=C) $cm^{-1}$; $^1H$ NMR (300 MHz, $CDCl_3$): δ 7.42 (d; H-6, $J_{6,7}$ = 6 Hz), 7.36-7.29 (m, 5H, Ph), 6.08 (dd, H-7, $J_{5,7}$ = 2.1 Hz), 5.7 (d, H-1, $J_{1,2}$ = 3.6 Hz), 5.28 (br s, H-5), 4.65; 4.61, 4.47 and 4.43 (AB system, $OCH_2Ph$, $J_{AB}$ = 12 Hz), 4.51 (dd, H-2, $J_{2,3}$ = 4.2 Hz), 4.33 (dd, H-4, $J_{4,5}$ = 3.3 Hz, $J_{3,4}$ = 9 Hz), 3.62 (dd, H-3), 1.59 (s, 3H, Me, isopropyl), 1.35 (s, 3H, Me, isopropyl); $^{13}C$ NMR (75,43 MHz, $CDCl_3$): δ 173.8 (C=O), 152.9 (C-6), 136.4 (Cq, Ph), 128.6; 128.3 (Ph), 121.9 (C-7), 112.0 (Cq, isopropyl), 104.3 (C-1), 86.8 (C-5), 81.5 (C-4), 78.5 (C-2), 72.2 ($OCH_2Ph$), 68.0 (C-3), 26.5 (Me, isopropyl), 26.3 (Me, isopropyl). Anal. calcd for $C_{18}H_{20}O_6$ (332.33): C 65.06; H 6.06; Found: C 65.05; H 6.29%.

Example 4

Preparation of 3-*O*-benzyl-6,7-dideoxy-1,2-*O*-isopropylidene-α-D-*gluco*-oct-6-enefuranurono-8,5-lactone **(4)**

Following a procedure similar to that of Example 1, starting from (7*R*)- and (7*S*)-3-*O*-benzyl-6,7-dideoxy-1,2-*O*-isopropylidene-7-phenylselenyl-α-D-*gluco*-octofuranurono-8,5-lactone[13,15] (112 mg, 0,23 mmol) the title compound was obtained (57,4 mg, 75%). $R_f$ 0.32 (ethyl acetate/*n*-hexane 1:4); m.p. = 66-70°C; $[\alpha]_D^{20}$ (c 0,5; $CHCl_3$); IR ($CHCl_3$): 1758 (C=O), 1380 (C-O, isopropyl) $cm^{-1}$; $^1H$ NMR (300 MHz, $CDCl_3$): δ 7.71 (dd, H-6, $J_{6,7}$ = 5.9 Hz), 7.37-7.31 (m, 5H, Ph), 6.15 (dd, H-7, $J_{5,7}$ = 1.5 Hz), 5.92 (d, H-1, $J_{1,2}$ = 3.5 Hz), 5.30 (dd, H-5, $J_{5,6}$ = 1.5 Hz), 4.73 - 4.65 (AB system, $OCH_2Ph$, $J_{AB}$ = 11 Hz), 4.63 (d, 1H, H-2), 4.15 (d, H-3, $J_{3,4}$ = 3.5 Hz), 3.97 (dd, H-4, $J_{4,5}$ = 6.7 Hz), 1.29 (s, 3H, Me, isopropyl), 1.43 (s, 3H, Me, isopropyl); $^{13}C$ NMR (75.43 MHz, $CDCl_3$): δ 172.7 (C=O), 156.5 (C-6), 137.0 (Cq, Ph), 128.6; 128.3; 128.1 (Ph), 121.7 (C-7), 112.3 (Cq, isopropyl), 105.4 (C-1), 82.3 (C-2), 81.8 (C-3), 81.2 (C-4), 79.2 (C-5), 72.9 ($OCH_2Ph$), 26.8 (Me, isopropyl), 26.2 (Me, isopropyl). Anal. calcd for $C_{18}H_{20}O_6$(332.33): C 65.06; H 6.06; Found: C 64.79; H 6.20%.

Example 6

Preparation of methyl (7*R*)-/(7*S*)-2,3-anhydro-6,7-dideoxy-7-methyl-7-phenylselenyl-β-L-*gulo*-octofuranurono-8,5-lactone **(6A/6B)**

A solution of *n*-BuLi (1,6 M in *n*-hexane, 5.43 mL, 8.7 mmol) was added dropwise to a solution of diisopropylamine (1.22 mL, 8.7 mmol) in anhydrous tetrahydrofuran (16 mL) under argon atmosphere at 0°C, and the mixture was stirred at 0°C for 25 min. A solution of phenylselenoacetic, phenylselenopropionic or phenylthioacetic acid (3.9 mmol) in anhydrous tetrahydrofuran (4 mL) was added dropwise to the reaction mixture, keeping the temperature at 0°C and the mixture was stirred for 1 h at 0°C. A solution of methyl 2,3;5,6-dianhydro-β-L-gulofuranoside (compound of Example 9, 624 mg, 3.95 mmol) in anhydrous tetrahydrofuran (3 mL/g of compound of Example 9) was then added dropwise and the reaction mixture was stirred first at 0°C for 1 h and then at room temperature for 16 h. After addition of a solution of 50% acetic acid (10 mL) and heating under reflux for six hours, the mixture was cooled to room temperature and neutralised with a saturated solution of $NaHCO_3$. After extraction of the mixture with diethyl ether (3x20 mL), the combined organic phases were washed with water and dried over sodium sulphate. Evaporation of the solvent under vacuum and purification by low pressure column chromatography, gave the title compounds (460 mg, 32%, **6A/6B:** 3/1). $R_f$ = 0.23 (ethyl acetate/*n*-hexane 1:1); IR (neat): 1784 (C=O), 1286 (C-O, epoxide) $cm^{-1}$; $^1H$ NMR (300 MHz, $CDCl_3$) of **6A:** δ 7.68-7.65 (m, 2H, Ph), 7.48-7.36 (m, 3H, Ph), 5.08 (s, 1H, H-1), 4.77 (ddd, 1H, H-5, $J_{4,5}$ = 6,9 Hz, $J_{5,6a}$ = 5.7 Hz, $J_{5,6b}$ =10.5 Hz), 4.02 (d, 1H, H-4, $J_{34}$ = 6.6 Hz), 3.74 (d, 1H, H-3, $J_{2,3}$ = 2.7 Hz), 3.68 (d, 1H, H-2), 3.55 (s, 3H, $OCH_3$), 2.52 (dd, 1H, H-6a , $J_{6a,6b}$ = 14.1 Hz), 2.32 (dd, 1H, H-6b), 1.66 (s, 3H, Me); $^{13}C$ NMR (75.43 MHz, $CDCl_3$) of **6B:** δ 176.0 (C-8), 137.7 (Cq, Ph), 129.7; 128.9 (Ph), 102.2 (C-1), 77.0 (C-4), 75.7 (C-5), 56.8 (OMe), 54.7 (C-2), 53.4 (C-3), 44.5 (C-7), 39.0 (C-6), 23.9 (Me). Anal. calcd for $C_{16}H_{18}O_5Se$ (369.25): C 52.04; H 4.90; Found: C 51.78; H 4.95%.

Example 7

Preparation of (7*R*)-/(7*S*)-3,6,7-Trideoxy-1,2-*O*-isopropylidene-7-methyl-7-phenylselenyl-α-D-*ribo*-octofuranurono-8,5-lactone **(7A/7B)**

Following a procedure similar to that of Example 6, starting from 5,6-anhydro-3-deoxy-1,2-*O*-isopropylidene-α-D-*ribo*-hexofuranose[15] (380 mg, 2.04 mmol), the title compounds were obtained (470 mg, 58%, **7A/7B:** 7/3), after separation by column chromatography with ethyl acetate/*n*-hexane (1:3) as eluent; $R_f$ = 0.27 (ethyl acetate/*n*-hexane (1:3); IR (KBr): 1754 (C=O), 1378 (CO, isopropyl) $cm^{-1}$; $^1H$ NMR (300 MHz, $CDCl_3$) of **7A**: δ 7.74-7.67 (m, 2H, Ph), 7.49-7.29 (m, 3H, Ph), 5.84 (d, 1H, H-1, $J_{1,2}$ = 3.3 Hz), 4.78 (t, 1H, H-2, $J_{2,3b}$ = 3.9 Hz), 4.48-4.41 (ddd, 1H, H-5, $J_{4,5}$ = 5.1 Hz, $J_{5,6b}$ = 10.1 Hz), 4.31-4.24 (ddd, 1H, H-4), 2.52 (dd, 1H, H-6a, $J_{5,6a}$ = 5.5 Hz , $J_{6a,6b}$ = 14.1 Hz), 2.27-2.14 (m, 2H, H-6b, H-3a), 1.73-1.66 (m, 4H, H-3b, Me), 1.53 (s, 3H, Me), 1.35 (s, 3H, Me). $^{13}C$ NMR (75,43 MHz, $CDCl_3$) of **7B:** δ 176.4 (C=O), 137.8 (Cq, Ph), 129.9; 129.1 (Ph), 111.6 (Cq, isopropyl), 105.6 (C-1), 80.2 (C-2), 78.6 (C-4), 76.9 (C-5), 44.7 (C-7), 40.5 (C-6), 35.1 (C-3), 26.7 (Me), 26.1 (Me), 24.0 (Me). Anal. calcd for $C_{18}H_{22}O_5Se$ (397.30): C 54.41; H 5.57; Found: C 54.79; H 5.72%.

Example 8

Preparation of (7*R*)-/(7*S*)-3-*O*-Benzyl-6,7-dideoxy-1,2-*O*-isopropylidene-7-methyl-7-phenylselenyl-α-D-*gluco*-octofuranurono-8,5-lactone **(8A/8B)**

Following a procedure similar to that of Example 6, starting from 5,6-anhydro-3-*O*-benzyl-1,2-*O*-isopropylidene-α-D-glucofuranose (430 mg, 1.47 mmol), the title compounds were obtained (590 mg, 79%, **8A/8B**:1/2), after separation by chromatography with ethyl acetate/*n*-hexane (1:6) as eluent; $R_f$ = 0,49 (ethyl acetate/*n*-hexane 1:6); IR (neat): 1773 (C=O), 1382 (C-O, isopropyl) $cm^{-1}$; $^1H$ NMR (300 MHz, $CDCl_3$): δ 7.67-7.63 (m, 4H, Ph), 7.41-7.26 (m, 16H, Ph), 5.95 (d, 1H, H-1**A**, $J_{1,2}$ = 3.6 Hz), 5.90 (d, 1H, H-1**B**, $J_{1,2}$ = 3.6 Hz), 4.83-4.17 (m, 7H, $OCH_2Ph$, H-2, H-5**A**), 4.18 (dd, 1H, H-4**A**, $J_{3,4}$ = 10.2 Hz, $J_{4,5}$ = 3.3 Hz), 4.09-4.05 (m, 2H, H-3), 3.78-3.75 (dd, 1H, H-4**B**, $J_{3,4}$ = 7.2 Hz, $J_{4,5}$ = 3.3 Hz), 3.16 (d, 1H, H-5**B**, $J_{5,6}$ = 3.9 Hz), 2.94 (dd, 1H, H-6a**B**, $J_{5,6a}$ = 3.9 Hz, $J_{6a,6b}$ = 5.1 Hz), 2.79 (dd, 1H, H-6b**B**, $J_{5,6b}$ = 2.4 Hz), 2.59 (dd, 1H, H-6aA, $J_{5,6a}$ = 5.7 Hz $J_{6a,6b}$ = 14.4 Hz), 2.38 (dd, 1H, H-6b**A**, $J_{5,6b}$ = 9.9 Hz), 1.63 (s ,6H, Me-7), 1.51 (s, 6H, Me, isopropyl), 1,31 (s, 6H, Me, isopropyl); $^{13}C$ NMR (75,43 MHz, $CDCl_3$): δ 176.7 (C=O), 137.6 (CqPh), 129.8; 129.0; 128.5; 128.1; 127.8; 127.6 (Ph), 112.1 (Cq-isopropyl), 105.2 (C-1), 82.6 (C-2), 82.5, 82.0 (C-3), 81.7, 81.5 (C-4), 73.1 (C-5**A**), 72.6 ($OCH_2Ph$), 48.2 (C-5**B**), 46.9 ($CH_2$-6**A**), 45.0, 44.2 (C-7), 41.3 ($CH_2$-6**B**), 26.8 (Me, isopropyl), 26.2 (Me, isopropyl), 24.0 (Me-7). Anal. calcd for $C_{25}H_{28}O_6Se$ (503.42): C 59.65; H 5.60; Found: C 60.00; H 5.93%.

**Biological Activity of the Compounds**

**Materials and Methods for Determination of Biological Activity**

A range of arthropod species was chosen to represent those in the terrestrial, aerial and aquatic environment, covering important target pest groups such as house and fruit flies and the white fly, which belongs to a group of agricultural and horticultural pests.

Method A

Topical treatment of adult fruit fly *(D. melanogaster*)

A culture of fruit flies (*D. melanogaster*) was used in the production of adult flies (approx 0.22 mg) of about seven days.

Serial dilutions of the compounds were prepared in acetone, and volumes of approximately 0.2 μL were applied to the ventral surface of each insect, using a calibrated PAX 100 microapplicator and a 1 mL syringe.

The fruit flies, in groups of 5, were anaesthetised using carbon dioxide. Prior to recovery the flies were placed in a containing vial and kept at 30±1° C, for observations of mortality at 1, 2, 3 and 24 hours after treatment.

For this purpose solutions of 6 different concentrations and a control were employed in groups of 12 and 20 insects. Control mortalities were normally zero but occasionally rose to 5-10%.

Method B

Second method for topical treatment of adult fruit flies

Different dilutions of the compounds in acetone were prepared and applied to individual fruit flies using a Gilson piston micropipette.

Individual flies were held in padded forceps and 1 μL of acetone solution was applied. The acetone was allowed to evaporate before placing the fly into the vial for observation, kept at 30±1° C, following the standard methodology.

Method C

Method by feeding adult fruit fly (*D. melanogaster*)

Large glass vials were used which were fitted with caps, inside of which is inserted a piece of cotton wool. This was soaked in a 10% sugar solution containing the test compounds in a given concentration.

Care was taken to ensure that no solution dripped from the cotton wool and condensation was avoided by keeping the vials at room temperature (25° C).

The fruit flies were anaesthetised using carbon dioxide and placed in the vials for recovery and feeding.

Method D

Topical treatment of fruit fly larvae

Fruit fly larvae were separated from the growing medium and second and third instars were topically treated with 0.2 μL acetone solutions of the compounds using a PAX microapplicator.
Larvae were held in padded forceps for dosing and then placed on moistened filter paper, at 30±1°C, for observation.

Method E

Topical treatment of house fly adults (*M. domestica*)

A culture of an insecticide-susceptible strain, Cooper, was established. Three-day-old adult house flies (body weight about 1.8 mg) were anaesthetised with carbon dioxide. Using the PAX microapplicator, a volume of 1 μL of acetone solution of the test compounds was applied to the dorsal cuticle, holding the fly in padded forceps.
Groups of flies were placed into closed vials, kept at 30 ±1° C, for observation.

Method F

Immersion bioassay of brine shrimp *A. salina* larvae in brine

Freshly hatched brine shrimps were prepared by adding aquarium brine shrimp eggs to salt water (15 g sea salt per litre water). The following day hatchlings were separated from eggs and empty egg cases, using their phototropic movement and a Pasteur pipette.
Into each of a set of small glass vials was pipetted 195 μL of brine containing 5 shrimp larvae, using a micropipette. The solution of the test compound in 5 μL acetone was added, the vial closed and kept at 30±1° C for observation.
Dead and alive shrimps were counted, at 1 and 24 hours after treatment, using a microscope. Six concentrations and a control were used with 10 shrimps treated.
A blank test was performed for comparison of the results.

Method G

Foliar treatment of glasshouse white fly (*T. vaporariorum*)

Seedlings of tomato plants were infested with adult white fly. Selected leaves of the tomato plants were excised and carefully trimmed to 3 leaflets without disturbing the infestation. These were placed in glass tubes containing water and the leaflets were then sprayed on both sides with a small sprayer delivering for each one 200 μL of a solution of the test compound dissolved in 30% acetone in water.
Controls were sprayed with the solvent alone.
Counts of insects on the individual leaflets were made immediately after spraying and then at 14 hours following.

Calculation of toxicity parameters

Dosages used in insect treatments were based upon the amount of compound applied to each insect. For the shrimps the final concentrations of the compounds in the immersion brine were used.
The 24 hour mortalities were used to calculate the $LD_{50}/LC_{50}$ using regression analysis of the probability percent mortality (probit) against log dose/concentration.[30] This was calculated using PoloPC software (LeOra Software, Berkeley, CA, 1994).
Where single dose treatments were used (in the case of whitefly assays), no statistics are available and the results are expressed as percent effect.

**Results**

Toxicity to fruit fly (*D. Melanogaster*)

The results of assays with fruit fly *D. Melanogaster* are given in Table 1.
In the table are also given the confidence intervals at 95% for the $LD_{50}$ values, the number of organisms tested,

the slope obtained by linear regression and the index g (of significance). Data are considered satisfactory if g is substancially less than 1 and seldom greater than 0.4.[31]

As regards the confidence intervals for $LD_{50}$, the indication (90%) means that the intervals were calculated at 90% and not at 95%.

From the analysis of the $LD_{50}$ values, calculated through method 1, it is found that in general the compounds tested are active against adult fruit flies, compounds of Examples 1, 2 and 3 being extremely active.

All of them are more active than imidacloprid, the reference insecticide for fruit fly.

Table 1 - Toxicity parameters of compounds tested on fruit flies according to methods A, B and D.

| Compound No. | $LD_{50}$ (μg/insect) | Confidence Intervals at 95% for $LD_{50}$ (μg/insect) | No. of Organisms Tested | Slope | g |
|---|---|---|---|---|---|
| **Method A** | | | | | |
| **1** | 0.00002 | 0.00000-0.00011 | 124 | 0.500±0.124 | 0.238 |
| **2** | $2.27x10^6$ | 0.00000-0.00002 | 150 | 0.308±0.066 | 0.175 |
| **3** | $5.02x10^{-6}$ | 0.00000-0.00008 | 114 | 0.340±0.104 | 0.362 |
| **4** | 0.00012 | n.d. | 114 | 0.385±0.109 | 0.860 |
| **6** | 0.00015 | 0.00000-0.00092 | 114 | 0.696±0.155 | 0.484 |
| **7** | 0.00020 | 0.00000 - 0.00154 (90%) | 114 | 0.395±0.104 | 0.527 |
| **8** | 0.00037 | n.d. | | 0.453±0.115 | 0.967 |
| **Imidacloprid** | 0.01253 | 0.00523-0.01637 | 75 | 2.218±0.911 | 0.648 |
| **Method B** | | | | | |
| **2** | 0.00398 | 0.00000-0.2415 (90%) | 48 | 0.484±0.206 | 0.693 |
| **Method D** | | | | | |
| **7** | 0.96796 | 0.72391-2.06494 | 75 | 2.624±0.810 | 0.366 |
| n.d.- Data not available | | | | | |

The fruit fly larvae are less sensitive to the toxins than adult flies.

The value of $LD_{50}$ obtained for the compound of Example 8 is much higher than that obtained for the adult fly, therefore this compound is less toxic for larvae than for adult flies.

Toxicity to house fly *M. domestica*

Toxicity parameters for adult house flies, treated topically with the compound of Examples 8, are given in Table 2.

Although these insects are approximately 8 fold larger than fruit flies, analysis of said table allows to conclude that the compounds tested are much less toxic (2 to 3 orders of magnitude) for this type of flies than for fruit flies.

$LD_{50}$ values correspond only to a moderate insecticidal activity.

Table 2 - Toxicity parameters of compounds tested on adult house flies

| Compound No. | $LD_{50}$ (μg/insect) | Confidence Intervals at 95% for $LD_{50}$ (μg/insect) | No. of Organisms Tested | Slope | g |
|---|---|---|---|---|---|
| **Method E** | | | | | |
| **8** | 0.64404 | n.d. | 30 | 0.383±0.182 | 0.863 |

Toxicity to brine shrimp

Toxicity parameters for assays in which brine shrimp larvae are exposed to the compounds in saline solution are given in Table 3. Data obtained show good correlation (g < 0,4) and high $LC_{50}$ values, indicating a low toxicity for this type of organisms.

Table 3 - Toxicity parameters of compounds tested on brine shrimp larvae, method F.

| **Compound** No. | **$LC_{50}$ (μg/mL)** | **Confidence Intervals at 95% for $LC_{50}$ (μg/mL)** | **No. of Organisms Tested** | **Slope** | **g** |
|---|---|---|---|---|---|
| **Method F** | | | | | |
| **1** | 100.62 | 90.03-125.27 | 50 | 8.862±2.644 | 0.342 |
| **2** | 64.30 | 57.75-77.28 | 50 | 9.217±2.638 | 0.315 |
| **3** | 144.71 | 128.12-172.71 | 50 | 7.763±2.182 | 0.303 |
| **4** | 358.92 | 324.89-400.42 | 60 | 9.770±2.057 | 0.170 |
| **6** | 125.48 | 113.08-139.97 | 50 | 10.275±2.421 | 0.213 |
| **7** | 261.04 | 220.59 - 320.09 (90%) | 50 | 8.601±2.195 | 0.778 |
| **8** | 220.41 | 199.24-249.71 | 50 | 10.359±2.659 | 0.253 |
| **Imidacloprid** | 0.03121 | 0.02387-0.04593 | 100 | 2.435±0.802 | 0.417 |

Insecticidal effect on adult white fly

The results of the bioassays of the insecticidal compounds on adult whiteflies *T. vaporariorum* are given in Table 4. Four compounds were tested, which were applied spraying 600 μL of each compound solution (prepared according to method G) on the leaves of tomato plants infested with a known number of adult whitefly. After 14 hours, the number of dead insects (or eventually of insects that disappeared) was counted. These assays were performed at room temperature, between 20 and 25°C.

From the data in Table 4, it is found that the compound of Examples 4 shows efficacy as insecticide.

Table 4 - Insecticidal effect againts adult white fly, assayed by spraying infested tomato leaves.

| **Compound No.** | **Concentration (μg/mL)** | **% Control of White Fly** |
|---|---|---|
| 3 | 1.3 | 0 |
| 4 | 2.5 | 50 |
| 6 | 1.3 | 0 |
| **7** | 4.8 | 0 |

**Discussion and conclusions**

Bioassays were performed using a range of compounds and treatment techniques in different species of arthropods. Adult fruit flies, treated topically, showed high levels of sensitivity to the compounds tested, such that the $LD_{50}$ values determined are much lower than that for the reference insecticide "imidacloprid". However some variation was observed in the toxicity effect produced by the different compounds.

The slope of the log-probit regression line was generally small and much smaller than that for imidacloprid. When treated by incorporation into the adult diet the compounds were much less toxic. Topical treatment of the larval stage was also much less toxic but the regression line slope increased uniformly.

Some of the compounds were tested by topical application on adult house fly and were much less toxic compared to the fruit fly adults.

The slopes of the log-probit regression line were similar to those obtained by the same type of treatment used in adult fruit fly, suggesting a mechanism of action similar although with less activity and some selectivity.

Contrary to the high insecticidal activity found, the compounds have a very low toxicity against brine shrimps.

The high values of $LC_{50}$ are associated with steep regression lines.

It can be concluded that these compounds show a very low toxicity towards this type of organisms in saline medium, not producing toxicity in these ecosystems.

In the test of spraying the compounds on leaves infested with adult white flies the compound of Example 4 was found to be promising for activity against the white fly. This compounds also showed high toxicity against adult fruit flies.

**References**

1. Haynes, L. J.; Plimmer, J. R Q. Rev. Chem. Soc. 1960, 14, 292-315.

2. Devon, T. K.; Scott, A. I. Handbook of Naturally Occurring Compounds; Academic Press: New York, 1972; Vol. II, pp. 79-175 (quoted in Ref. 8).

3. Marshall, P. G. In Chemistry of Carbon Compounds; Rodd, E. H., Ed.; Elsevier: New York, 1970; Vol. II D, Chapter 17 (quoted in Ref. 8).

4. (a) Schmitz, F. J.; Kraus, K. W.; Ciereszko, L. S.; Sifford, D. H.; Weinheimer, A. J. Tetrahedron Lett. 1966, 7, 97-104; (b) Cimino, G.; De Stefano, S.; Minale, L.; Fattorusso, E. Tetrahedron 1972, 28, 333-341; (c) Cafieri, F.; Fattorusso, E.; Santacroce, C.; Minale, L.; Tetrahedron 1972, 28, 1579-1583; (d) Faulkner, D. J. Tetrahedron Lett. 1973, 14, 3821-3822; (e) Rothberg, I.; Shubiak, P. Tetrahedron Lett. 1975, 16, 769-722; (f) Cimino, G.; De Stefano, S.; Guerriero, A.; Minale, L. Tetrahedron Lett. 1975, 16, 1417-1420.

5. Ma, S.; Schi, Z. ; Yu, Z. Tetrahedron 1999, 55, 12137-12148.

6. Larock, R. C. ; Riefling, B.; Fellows, C. A. J. Org. Chem. 1978, 43, 131-137.

7. Brownbridge, P.; Chan, T. H. Tetrahedron Lett. 1980, 21, 3431-3434.

8. Cardellach, J.; Estopa, C.; Font, J.; Moreno-Manas, M.; Ortuño, R. M.; Sachez-Ferrando, F.; Valle, S.; Vilamajo, L. Tetrahedron 1982, 38, 2377-2394.

9. Kotora, M.; Negishi, E. Synthesis 1997, 121-128.

10. Klein Gebbinck, E. A.; Stork, G. A.; Jansen, B. J. M.; of Groot, A. Tetrahedron 1999, 55, 11077-11094.

11. Choudhury, P. K.; Foubelo, F.; Yus, M. Tetrahedron 1999, 55, 10779-10788.

12. Figueredo, M.; Font, J.; Virgili, A. Tetrahedron 1987, 43, 1881-1886.

13. Rauter, A. P.; Ferreira, M. J.; Font, J.; Virgili, A.; Figueredo, M.; Figueiredo, J. A.; Ismael, M. I.; Canda, T. L. J. Carbohydr. Chem. 1995, 14, 929-948.

14. Rauter, A. P.; Figueiredo, J. A.; Ismael, M. I.; Pais, M. S.; Gonzalez, A. G.; Dias, J.; Barrera, J. B. J. Carbohydr. Chem. 1987, 6, 259-272.

15. Rauter, A. P.; Figueiredo, J.; Ismael, M.; Canda, T. L.; Font, J.; Figueredo, M. Tetrahedron: Asymmetry 2001, 12, 1131-1146.

16. Csuk, R.; Furstner, A.; Weidmann, H. J. Chem. Soc., Chem. Commun. 1986, 775.

17. Csuk, R.; Glänzer, B. I. ; Hu, Z.; Boese, R. Tetrahedron 1994, 50, 1111-1124.

18. Hanessian, S.; Girard, C. Synlett 1994, 10, 865-867.

19. Rao, A. S. Tetrahedron 1983, 39, 2323-2367.

20. Garem, B. Tetrahedron 1978, 34, 3353-3383.

21. Rodrigues, J.; Dulcere, J. P. Synthesis, 1993, 1177-1202.

22. Waggins, L. F. Nature, 1950, 165.

23. Ohle, M.; Vargha, L. V. J. Chem. Soc. 1959, 2717.

24. Mitsunobu, O. Synthesis 1981, 1.

25. Szeja, W. Synthesis 1985, 983-985.

26. Kwart, H.; Hoffman, D. M. J. Org. Chem. 1966, 31, 419- 425.

27. Gutshe, C. D. Organic Reactions, 1954, 8, 364.

28. Rauter, A. P.; Figueiredo, J. A.; Ismael, M. I., Carbohydr. Res. 1989, 188, 19-24.

29. Dax, K.; Rauter, A. P.; Stütz, A. E.; Weidmann, H., Liebigs Ann. Chem. 1981, 1768-1773.

30. Robertson, J. L.; Preisher, H., Pesticide Bioassays with Arthropods, 1992, CRC Press, Boca Raton, FL.

31. Finney, D. J., Probit Analysis, 1972, 3rd Ed., Cambridge University Press, London, p. 79.

## Claims

**1.** The use, as an insecticide, of a compound of formula (I)

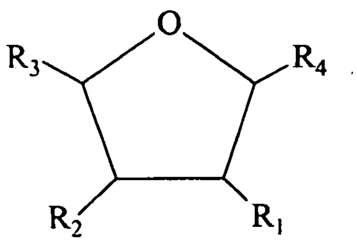

(I)

wherein

$R_1$ and $R_2$ represent, independently, hydrogen, halogen, alkoxy, substituted alkoxy or an ester group; or
$R_1$ and $R_2$, together with the carbon atoms to which they are attached, represent an oxirane ring; or
$R_1$ and $R_2$, taken together, represent an akylidenedioxy or substituted alkylidenedioxy group; and
$R_3$ represents a group of formula

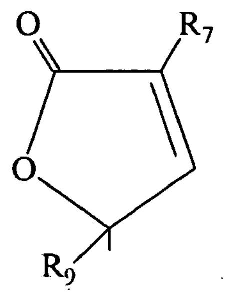

or

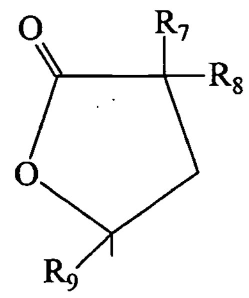

wherein

$R_7$ represents hydrogen or alkyl,
$R_8$ represents phenylsulfanyl, phenylselenyl, phenylsulfoxy or phenylselenoxy, and
$R_9$ represents hydrogen, ethoxycarbonyl or carbamoyl; and

$R_4$ represents, independently, hydrogen, alkoxy or substituted alkoxy, or
$R_1$ and $R_4$, taken together, represent an alkylidenedioxy or a substituted alkylidenedioxy group.

**2.** The use, according to claim 1, wherein, in the insecticidal compound of formula (I)

$R_1$ and $R_2$ or represent, independently, hydrogen, alkoxy, substituted alkoxy an ester group, or
$R_1$ and $R_2$, together with the carbon atoms to which they are attached, represent an oxirane ring; or
$R_1$ and $R_2$, taken together, represent an alkylidenedioxy or a substituted alkylidenedioxy group; and
$R_3$ represents a group of formula

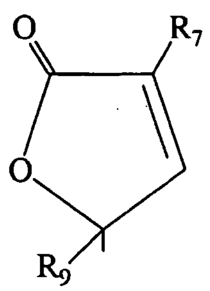

wherein

$R_7$ represents hydrogen or alkyl, and
$R_9$ represents hydrogen, ethoxycarbonyl or carbamoyl; and

$R_4$ represents, independently, hydrogen, alkoxy or substituted alkoxy, or
$R_1$ and $R_4$, taken together, represent an alkylidenedioxy or substituted alkylidenedioxy group.

**3.** The use, according to claim 2, wherein the insecticidal compound of formula (I) presents the formula (IA)

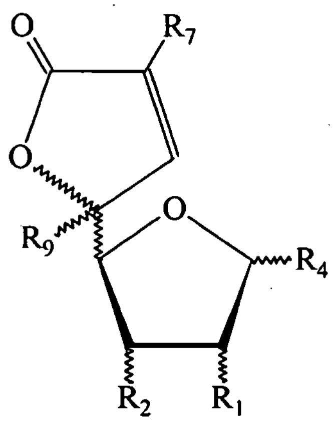

(IA)

wherein $R_1$, $R_2$, $R_4$, $R_7$ and $R_9$ are as defined in claim 2.

**4.** The use, according to claim 3, wherein $R_1$ and $R_4$, taken together, represent an isopropylidenedioxy group, $R_2$ represents benzyloxy, $R_7$ represents methyl and $R_9$ represents hydrogen.

**5.** The use, according to claim 4, wherein the compound is a D-*gluco* derivative, *i.e.* 3-*O*-benzyl-6,7-dideoxy-1,2-*O*-isopropylidene-7-methyl-α-D-*gluco*-oct-6-enefuranurono-8,5-lactone.

**6.** The use, according to claim 4, wherein the compound is a D-*alo* derivative, *i.e.* 3-*O*-benzyl-6,7-dideoxy-1,2-*O*-isopropylidene-7-methyl-α-D-*alo*-oct-6-enefuranurono-8,5-lactone.

**7.** The use, according to claim 3, wherein $R_1$ and $R_4$, taken together, represent an isopropylidenedioxy group, $R_2$ represents benzyloxy and $R_7$ and $R_9$ represents hydrogen.

**8.** The use, according to claim 7, wherein the compound is a D-*alo* derivative; *i.e.* 3-*O*-benzyl-6,7-dideoxy-1,2-*O*-isopropylidene-α-D-*alo*-oct-6-enefuranurono-8,5-lactone.

**9.** The use, according to claim 7, wherein the compound is a D-*gluco* derivative, *i.e.* a 3-*O*-benzyl-6,7-dideoxy-1,2-*O*-isopropylidene-α-D-*gluco*-oct-6-enefuranurono-8,5-lactone.

**10.** The use, according to claim 1, wherein, in the insecticidal compound of formula (I)

$R_1$ and $R_2$ represent, independently, hydrogen, alkoxy or substituted alkoxy, or
$R_1$ and $R_2$, together with the carbon atoms to which they are attached, represent an oxirane ring; or
$R_1$ and $R_2$, taken together, represent an alkylidenedioxy or substituted alkylidenedioxy group; and
$R_3$ represents a group of formula

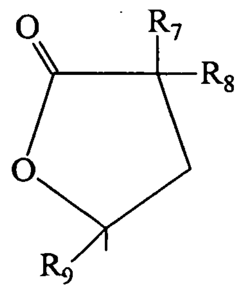

wherein

$R_7$ represents hydrogen or alkyl,
$R_8$ represents phenylsulfanyl, phenylselenyl, phenylsulfoxy or phenylselenoxy, and
$R_9$ represents hydrogen, ethoxycarbonyl or carbamoyl; and

$R_4$ represents, independently, hydrogen, alkoxy or substituted alkoxy, or
$R_1$ and $R_4$, taken together, represent an alkylidenedioxy or substituted alkylidenedioxy group.

**11.** The use, according to claim 10, wherein the insecticidal compound of formula (I) presents the formula (IC)

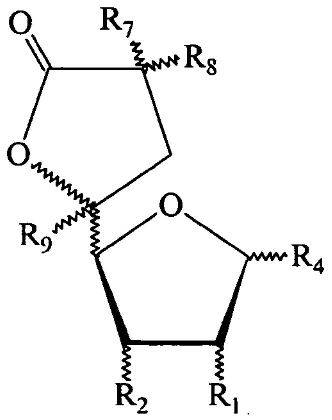

(IC)

wherein $R_1$, $R_2$, $R_4$, $R_7$, $R_8$ and $R_9$ are as defined in claim 10.

**12.** The use, according to claim 11, wherein $R_1$ and $R_2$, taken together with the carbon atoms to which they are attached, form an oxirane ring, $R_4$ represents methoxy, $R_7$ represents methyl, $R_8$ represents phenylselenyl and $R_9$ represents hydrogen.

**13.** The use, according to claim 12, wherein the compound is a 2,3-anhydro-β-L-*gulo* derivative, *i.e.* methyl (7*R*)- and methyl (7*S*)-2,3-anhydro-6,7-dideoxy-7-methyl-7-phenylselenyl-β-L-*gulo*-octofuranurono-8,5-lactone.

**14.** The use, according to claim 11, wherein $R_1$ and $R_4$, taken together, form an isopropylidenedioxy group, $R_2$ represents hydrogen, $R_7$ represents methyl, $R_8$ represents phenylselenyl and $R_9$ represents hydrogen.

**15.** The use, according to claim 14, wherein the compound is a D-*ribo* derivative, *i.e.* (7*R*)- and (7*S*)-3,6,7-trideoxy-1,2-*O*-isopropylidene-7-methyl-7-phenylselenyl-α-D-*ribo*-octofuranurono-8,5-lactone.

**16.** The use, according to claim 11, wherein $R_1$ and $R_4$, taken together, form an isopropylidenedioxy group, $R_2$ represents benzyloxy, $R_7$ represents methyl, $R_8$ represents phenylselenyl and $R_9$ represents hydrogen.

**17.** The use, according to claim 16, wherein the compound is a D-*gluco* derivative, *i.e.* a (7*R*)- and (7*S*)-3-*O*-benzyl-6,7-dideoxy-1,2-*O*-isopropylidene-7-methyl-7-phenylselenyl-α-D-*gluco*-octofuranurono-8,5-lactone.

**18.** The use, according to any one of claims 1 to 17, wherein the insects are fruit fly (*Drosophila melanogaster*), house fly (*Musca domestica*) and white fly (*Trialeurodes vaporarium*).

**19.** A method for controlling insects, comprising the application of an effective amount of compounds of formula (I) to said insects or their locus.

**20.** The method, according to claim 19, wherein the insects are fruit fly (*Drosophila melanogaster*), house fly (*Musca domestica*) and white fly (*Trialeurodes vaporarium*).

## Patentansprüche

**1.** Der Gebrauch als Insektizid einer Verbindung der Formel (I),

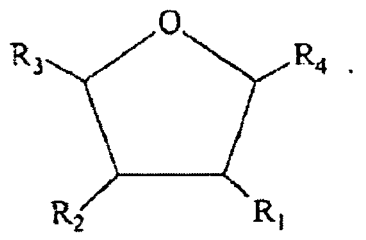

(I)

wobei
R1 und R2 für sich Wasserstoff, Halogen, eine Alkoxy-, eine substituierte Alkoxy- oder eine Estergruppe darstellen, oder
R1 und R2 zusammen mit den Kohlenstoffatomen, mit denen sie verknüpft sind, einen Oxiranring darstellen, oder
R1 und R2 zusammengenommen eine Alkylidendioxy- oder substitutierte Alkylidendioxygruppe darstellen, oder
R3 eine Gruppe der Formeln

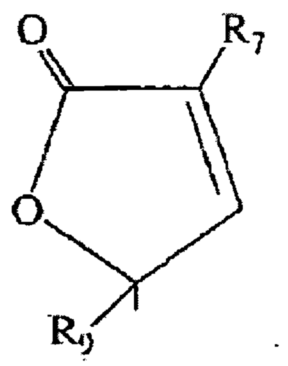

oder

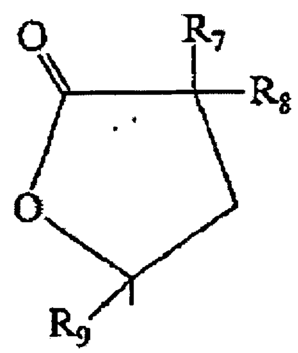

darstellt, wobei
R7 Wasserstoff oder Alkyl darstellt,
R8 Phenylsulfanyl, Phenylselenyl, Phenylsulfoxy oder Phenylselenoxy darstellt,
R9 Wasserstoff, Ethoxycarbonyl oder Carbamoyl darstellt; und
R4 für sich Wasserstoff, Alkoxy oder substituiertes Alkoxy darstellt oder
R1 und R4 zusammengenommen eine Alkylidendioxy- oder eine substituierte Alkylidendioxygruppe darstellen.

**2.** Die Verwendung nach Patentanspruch 1, wobei in der insektiziden Verbindung der Formel (I)
R1 und R2 für sich Wasserstoff, Halogen, eine Alkoxy-, eine substituierte Alkoxy- oder eine Estergruppe darstellen,
R1 und R2 zusammen mit den Kohlenstoffatomen, mit denen sie verknüpft sind, einen Oxiranring darstellen, oder
R1 und R2 zusammengenommen eine Alkylidendioxy- oder substitutierte Alkylidendioxygruppe darstellen, oder
R3 eine Gruppe der Formel

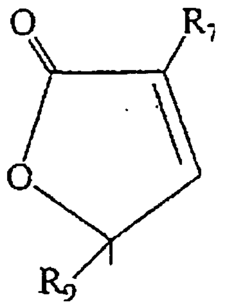

darstellt, wobei
R7 Wasserstoff oder Alkyl darstellt, und
R9 Wasserstoff, Ethoxycarbonyl oder Carbamoyl darstellt, und
R4 für sich Wasserstoff, Alkoxy oder substituiertes Alkoxy darstellt oder
R1 und R4 zusammengenommen eine Alkylidendioxy- oder eine substituierte Alkylidendioxygruppe darstellen.

3. Die Verwendung nach Patentanspruch 2, wobei die insektizide Verbindung der Formel (I) durch die Formel (Ia) dargestellt wird

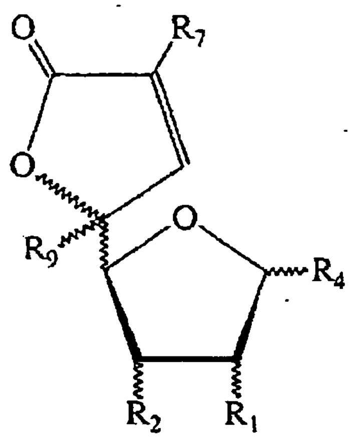

(Ia)

wobei R1, R2, R4, R7 und R9 der Definition in Patentanspruch 2 folgen.

4. Die Verwendung nach Patentanspruch 3, wobei R1 und R4 zusammengenommen eine Isopropylidendioxygruppe darstellen, R2 Benzyloxy darstellt, R7 Methyl darstellt und R9 Wasserstoff darstellt.

5. Die Verwendung nach Patentanspruch 4, wobei die Verbindung ein D-*gluco*-Derivat ist, d.h. 3-O-benzyl-6,7-dideoxy-1,2-isopropyliden-7-methyl-α-D-*gluco*-oct-6-enfuranurono-8,5-lacton.

6. Die Verwendung nach Patentanspruch 4, wobei die Verbindung ein D-*alo*-Derivat ist, d.h. 3-O-benzyl-6,7-dideoxy-1,2-O-isopropyliden-7-methyl-α-D-*alo*-oct-6-enfuranurono-8,5-lacton.

7. Die Verwendung nach Patentanspruch 3, wobei R1 und R4 zusammengenommen eine Isopropylidendioxygruppe darstellen, R2 Benzyloxy darstellt und R7 und R9 Wasserstoff darstellt.

8. Die Verwendung nach Patentanspruch 7, wobei die Verbindung ein D-*alo*-Derivat ist, d.h. 3-O-benzyl-6,7-dideoxy-1,2-O -isopropyliden--α-D-*alo*-oct-6-enfuranurono-8,5-lacton.

9. Die Verwendung nach Patentanspruch 7, wobei die Verbindung ein D-*gluco*-Derivat ist, d.h. 3-O-benzyl-6,7-dideoxy-1,2-O -isopropyliden--α-D-*gluco*-oct-6-enfuranurono-8,5-lacton.

**10.** Die Verwendung nach Patentanspruch 1, wobei in der insektiziden Verbindung der Formel (I)
R1 und R2 für sich Wasserstoff, Halogen, eine Alkoxy-, eine substituierte Alkoxygruppe darstellen, oder
R1 und R2 zusammen mit den Kohlenstoffatomen, mit denen sie verknüpft sind, einen Oxiranring darstellen, oder
R1 und R2 zusammengenommen eine Alkylidendioxy- oder substitutierte Alkylidendioxygruppe darstellen, oder
R3 eine Gruppe der Formel

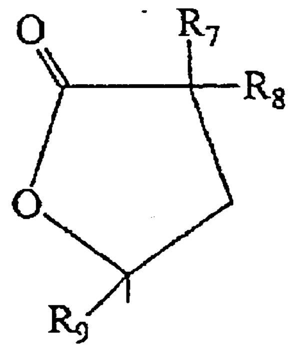

darstellt, wobei
R7 Wasserstoff oder Alkyl darstellt,
R8 Phenylsulfanyl, Phenylselenyl, Phenylsulfoxy oder Phenylselenoxy darstellt,
R9 Wasserstoff, Ethoxycarbonyl oder Carbamoyl darstellt; und
R4 für sich Wasserstoff, Alkoxy oder substituiertes Alkoxy darstellt oder
R1 und R4 zusammengenommen eine Alkylidendioxy- oder eine substituierte Alkylidendioxygruppe darstellen.

**11.** Die Verwendung nach Patentanspruch 10, wobei die insektizide Verbindung der Formel (I) durch die Formel (IC) dargestellt wird,

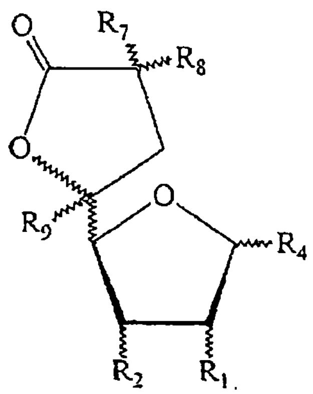

wobei R1, R2, R4, R7, R8 und R9 wie in Patentanspruch 10 definiert sind.

**12.** Die Verwendung nach Patentanspruch 11, wobei R1 und R2 zusammen mit den Kohlenstoffatomen, mit denen sie verknüpft sind, einen Oxiranring bilden, R4 Methoxy darstellt, R7 Methyl darstellt, R8 Phenylselenyl darstellt und R9 Wasserstoff darstellt.

**13.** Die Verwendung nach Patentanspruch 12, wobei die Verbindung ein 2,3-anhydro-β-L-*gulo*-Derivat ist, d.h. Methyl-(7R)- und Methyl-(7S)-2,3-anhydro-6,7-dideoxy-7-methyl-7-phenylselenyl-β-L-*gulo*-octofuranurono-8,5-lacton.

**14.** Die Verwendung nach Patentanspruch 11, wobei R1 und R4 zusammengenommen eine Isopropylidendioxygruppe bilden, R2 Methyl darstellt, R8 Phenylselenyl darstellt und R9 Wasserstoff darstellt.

**15.** Die Verwendung nach Patentanspruch 14, wobei die Verbindung ein D-*ribo*-Derivat ist, d.h. 7R)- und (7S)-3,6,7-trideoxy-1,2-O-isopropyliden-7-methyl-7-phenylselenyl-α-D-*ribo*-octofuranurono-8,5-lacton.

**16.** Die Verwendung nach Patentanspruch 11, wobei R1 und R4 zusammengenommen, eine Isopropylidendioxygruppe bilden, R2 Benzyloxy darstellt, R7 Methyl darstellt, R8 Phenylselenyl darstellt und R9 Wasserstoff darstellt.

**17.** Die Verwendung nach Patentanspruch 16, wobei die Verbindung ein D-*gluco*-Derivat ist, d.h. (7R)- und (7S)-3-O-benzyl-6,7-dideoxy-1,2-O-isopropyliden-7-methyl-7-phenylselenyl-α-D-*gluco*-octofuranurono-8,5-lacton.

**18.** Die Verwendung nach jedem der Patentansprüche 1 bis 17, wobei die Insekten Fruchtfliege (*Drosophila melanogaster*), Stubenfliege (*Musca domestica*) und Weiße Fliege (*Trialeurodes vaporarium*) sind.

**19.** Eine Methode zur Kontrolle von Insekten, die die Anwendung einer wirksamen Menge von Verbindungen der Formel (I) an besagte Insekten und deren Aufenthaltsort umfasst.

**20.** Die Verwendung nach Patentanspruch 19, wobei die Insekten Fruchtfliege (*Drosophila melanogaster*), Stubenfliege (*Musca domestica*) und Weiße Fliege (*Trialeurodes vaporarium*) sind.

## Revendications

**1.** Utilisation, comme insecticide, d'un composé de formule (I)

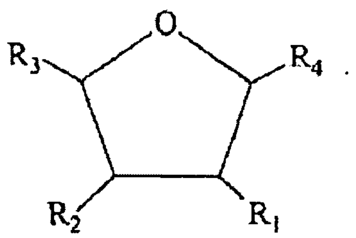

dans laquelle

$R_1$ et $R_2$ représentent, indépendamment, hydrogène, halogène, alcoxy, alcoxy substitué ou un groupe ester ; ou $R_1$ et $R_2$, conjointement avec les atomes de carbone auxquels ils sont liés, représentent un anneau oxyrane ; ou $R_1$ et $R_2$, pris conjointement, représentent un groupe alkylidènedioxy ou alkylidènedioxy substitué ; et $R_3$ représente un groupe de formule

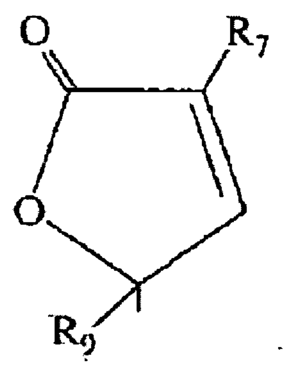

ou

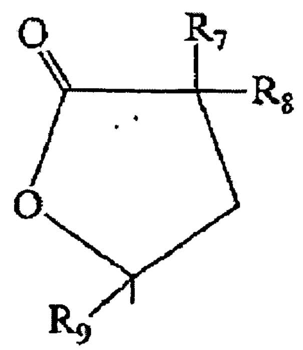

dans laquelle

$R_7$ représente hydrogène ou alkyle,
$R_8$ représente phénylsulphanyle, phénylsélényle, phénylsulphoxy ou phénylsélénoxy, et

$R_9$ représente hydrogène, éthoxycarbonyle ou carbamoyle ; et

$R_4$ représente, indépendamment, hydrogène, alcoxy ou alcoxy substitué, et
$R_1$ et $R_4$, pris conjointement, représentent un groupe alkylidènedioxy ou alkylidènedioxy substitué.

**2.** Utilisation selon la revendication 1, dans laquelle, dans le composé d'insecticide de formule (I)

$R_1$ et $R_2$ représentent, indépendamment, hydrogène, alcoxy, alcoxy substitué ou un groupe ester, ou
$R_1$ et $R_2$, conjointement avec les atomes de carbone auxquels ils sont liés, représentent un anneau oxyrane ; ou
$R_1$ et $R_2$, pris conjointement, représentent un groupe alkylidènedioxy ou alkylidènedioxy substitué ; et
$R_3$ représente un groupe de formule

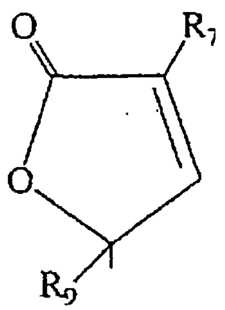

dans laquelle

$R_7$ représente hydrogène ou alkyle, et
$R_9$ représente hydrogène, éthoxycarbonyle ou carbamoyle ; et

$R_4$ représente, indépendamment, hydrogène, alcoxy ou alcoxy substitué, ou
$R_1$ et $R_4$, pris conjointement, représentent un groupe alkylidènedioxy ou alkylidènedioxy substitué.

**3.** Utilisation selon la revendication 2, dans laquelle le composé d'insecticide de formule (I) présente la formule (IA)

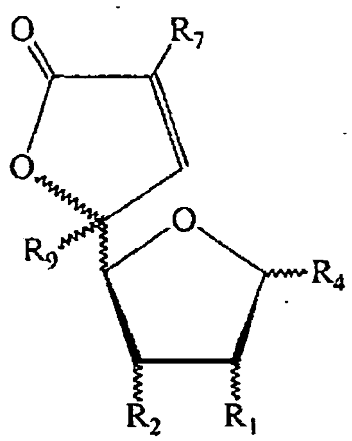

(IA)

dans laquelle $R_1$, $R_2$, $R_4$, $R_7$ et $R_9$ sont tels que définis dans la revendication 2.

**4.** Utilisation selon la revendication 3, dans laquelle $R_1$ et $R_4$, pris conjointement, représentent un groupe isopropylidènedioxy, $R_2$ représente benzyloxy, $R_7$ représente méthyle et $R_9$ représente hydrogène.

**5.** Utilisation selon la revendication 4, dans laquelle le composé est un dérivé D-*gluco*, c'est-à-dire, 3-*O*-benzyle-6,7-didéoxy-1,2-*O*-isopropylidène-7-méthyle-a-D-*gluco*-oct-6-énéfuranurono-8,5-lactone.

**6.** Utilisation selon la revendication 4, dans laquelle le composé est un dérivé D-*alo,* c'est-à-dire, 3-*O*-benzyle-6,7-didéoxy-1,2-*O*-isopropylidène-7-méthyle-α-D-*alo*-oct-6-énéfuranurono-8,5-lactone.

**7.** Utilisation selon la revendication 3, dans laquelle $R_1$ et $R_4$, pris conjointement, représentent un groupe isopropylidènedioxy, $R_2$ représente benzyloxy et $R_7$ et $R_9$ représentent hydrogène.

**8.** Utilisation selon la revendication 7, dans laquelle le composé est un dérivé D-*alo*, c'est-à-dire, 3-*O*-benzyle-6,7-didéoxy-1,2-*O*-isopropylidène-α-D-*alo*-oct-6-énéfuranurono-8,5-lactone.

**9.** Utilisation selon la revendication 7, dans laquelle le composé est un dérivé D-*gluco*, c'est-à-dire, 3-*O*-benzyle-6,7-didéoxy-1,2-*O*-isopropylidène-α-D-*gluco*-oct-6-énéfuranurono-8,5-lactone.

**10.** Utilisation selon la revendication 1, dans laquelle, dans le composé d'insecticide de formule (I)

$R_1$ et $R_2$ représentent, indépendamment, hydrogène, halogène, alcoxy ou alcoxy substitué, ou
$R_1$ et $R_2$, conjointement avec les atomes de carbone auxquels ils sont liés, représentent un anneau oxyrane ; ou
$R_1$ et $R_2$, pris conjointement, représentent un groupe alkylidènedioxy ou alkylidènedioxy substitué ; et
$R_3$ représente un groupe de formule

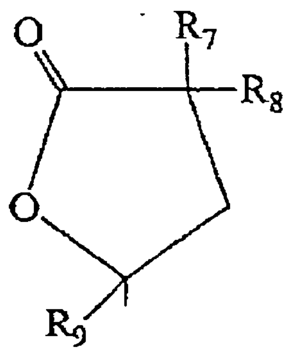

dans laquelle

$R_7$ représente hydrogène ou alkyle,
$R_8$ représente phénylsulphanyle, phénylsélényle, phénylsulphoxy ou phénylsélénoxy, et
$R_9$ représente hydrogène, éthoxycarbonyle ou carbamoyle ; et

$R_4$ représente, indépendamment, hydrogène, alcoxy ou alcoxy substitué ; et
$R_1$ et $R_4$, pris conjointement, représentent un groupe alkylidènedioxy ou alkylidènedioxy substitué.

**11.** Utilisation selon la revendication 10, dans laquelle le composé d'insecticide de formule (I) présente la formule (IC)

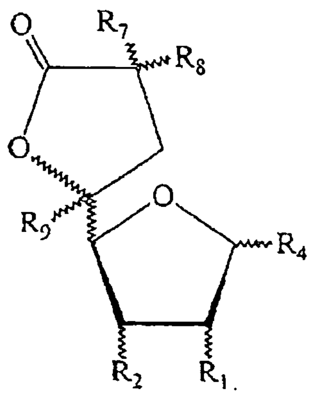

dans laquelle $R_1$, $R_2$, $R_4$, $R_7$, $R_8$ et $R_9$ sont tels que définis dans la revendication 10.

**12.** Utilisation selon la revendication 11, dans laquelle $R_1$ et $R_2$, pris conjointement avec les atomes de carbone auxquels ils sont liés, forment un anneau oxyrane, $R_4$ représente méthoxy, $R_7$ représente méthyle, $R_8$ représente phénylsélényle et $R_9$ représente hydrogène.

**13.** Utilisation selon la revendication 12, dans laquelle le composé est un dérivé 2,3-anhydro-β-L-*gulo*, c'est-à-dire, méthyle (7*R*)- et méthyle (7*S*)-2,3-anhydro-6,7-didéoxy-7-méthyle-7-phénylsélényle-β-L-*gulo*-octofuranurono-8,5-lactone.

**14.** Utilisation selon la revendication 11, dans laquelle $R_1$ et $R_4$, pris conjointement, forment un groupe isopropylidènedioxy, $R_2$ représente hydrogène, $R_7$ représente méthyle, $R_8$ représente phénylsényle et $R_9$ représente hydrogène.

**15.** Utilisation selon la revendication 14, dans laquelle le composé est un dérivé D-*ribo,* c'est-à-dire (7*R*)- et (7*S*)-3,6,7-tridéoxy-1,2-*O*-isopropylidène-7-méthyle-7-phénylsélényle-α-D-*ribo*-octofuranurono-8,5-lactone.

**16.** Utilisation selon la revendication 11, dans laquelle $R_1$ et $R_4$, pris conjointement, forment un groupe isopropylidènedioxy, $R_2$ représente benzyloxy, $R_7$ représente méthyle, $R_8$ représente phénylsényle et $R_9$ représente hydrogène.

**17.** Utilisation selon la revendication 16, dans laquelle le composé est un dérivé D-*gluco*, c'est-à-dire (7*R*)- et (7*S*)-3,*O*-benzyle-6,7-didéoxy-1,2-*O*-isopropylidène-7-méthyle-7-phénylsélényle-α-D-*gluco*-octofuranurono-8,5-lactone.

**18.** Utilisation selon l'une quelconque des revendications 1 à 17, dans laquelle les insectes sont la mouche des fruits (*Drosophila melanogaster*), la mouche domestique (*Musca domestica*) et la mouche blanche (*Trialeurodes vaporarium*).

**19.** Méthode pour contrôler les insectes, comprenant l'application d'une quantité efficace de composés de formule (I) auxdits insectes ou à leur locus.

**20.** Méthode selon la revendication 19, dans laquelle les insectes sont la mouche des fruits (*Drosophila melanogaster*), la mouche domestique (*Musca domestica*) et la mouche blanche (*Trialeurodes vaporarium*).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Haynes, L. J. ; Plimmer, J. R Q.** *Rev. Chem. Soc.,* 1960, vol. 14, 292-315
- **Devon, T. K. ; Scott, A. I.** Handbook of Naturally Occurring Compounds. Academic Press, 1972, vol. II, 79-175
- **Marshall, P. G.** Chemistry of Carbon Compounds. Elsevier, 1970, vol. II D
- **Schmitz, F. J. ; Kraus, K. W. ; Ciereszko, L. S. ; Sifford, D. H. ; Weinheimer, A. J.** *Tetrahedron Lett,* 1966, vol. 7, 97-104
- **Cimino, G. ; De Stefano, S. ; Minale, L. ; Fattorusso, E.** *Tetrahedron,* 1972, vol. 28, 333-341
- **Cafieri, F. ; Fattorusso, E. ; Santacroce, C. ; Minale, L.** *Tetrahedron,* 1972, vol. 28, 1579-1583
- **Faulkner, D. J.** *Tetrahedron Lett.,* 1973, vol. 14, 3821-3822
- **Rothberg, I. ; Shubiak, P.** *Tetrahedron Lett.,* 1975, vol. 16, 769-722
- **Cimino, G. ; De Stefano, S. ; Guerriero, A. ; Minale, L.** *Tetrahedron Lett.,* 1975, vol. 16, 1417-1420
- **Ma, S. ; Schi, Z. ; Yu, Z.** *Tetrahedron,* 1999, vol. 55, 12137-12148
- **Larock, R. C. ; Riefling, B. ; Fellows, C. A.** *J. Org. Chem,* 1978, vol. 43, 131-137
- **Brownbridge, P. ; Chan, T. H.** *Tetrahedron Lett.,* 1980, vol. 21, 3431-3434
- **Cardellach, J. ; Estopa, C. ; Font, J. ; Moreno-Manas, M. ; Ortuño, R. M. ; Sachez-Ferrando, F. ; Valle, S. ; Vilamajo, L.** *Tetrahedron,* 1982, vol. 38, 2377-2394
- **Kotora, M. ; Negishi, E.** *Synthesis,* 1997, 121-128
- **Klein Gebbinck, E. A. ; Stork, G. A. ; Jansen, B. J. M. ; Groot, A.** *Tetrahedron,* 1999, vol. 55, 11077-11094
- **Choudhury, P. K. ; Foubelo, F. ; Yus, M.** *Tetrahedron,* 1999, vol. 55, 10779-10788
- **Figueredo, M. ; Font, J. ; Virgili, A.** *Tetrahedron,* 1987, vol. 43, 1881-1886
- **Rauter, A. P. ; Ferreira, M. J. ; Font, J. ; Virgili, A. ; Figueredo, M. ; Figueiredo, J. A. ; Ismael, M. I. ; Canda, T. L.** *J. Carbohydr. Chem.,* 1995, vol. 14, 929-948
- **Rauter, A. P. ; Figueiredo, J. A. ; Ismael, M. I. ; Pais, M. S. ; Gonzalez, A. G. ; Dias, J. ; Barrera, J. B.** *J. Carbohydr. Chem.,* 1987, vol. 6, 259-272
- **Rauter, A. P. ; Figueiredo, J. ; Ismael, M. ; Canda, T. L. ; Font, J. ; Figueredo, M.** *Tetrahedron: Asymmetry,* 2001, vol. 12, 1131-1146
- **Csuk, R. ; Furstner, A. ; Weidmann, H.** *J. Chem. Soc., Chem. Commun.,* 1986, 775
- **Csuk, R. ; Glänzer, B. I. ; Hu, Z. ; Boese, R.** *Tetrahedron,* 1994, vol. 50, 1111-1124
- **Hanessian, S. ; Girard, C.** *Synlett,* 1994, vol. 10, 865-867
- **Rao, A. S.** *Tetrahedron,* 1983, vol. 39, 2323-2367
- **Garem, B.** *Tetrahedron,* 1978, vol. 34, 3353-3383
- **Rodrigues, J. ; Dulcere, J. P.** *Synthesis,* 1993, 1177-1202
- **Waggins, L. F.** *Nature,* 1950, 165
- **Ohle, M. ; Vargha, L. V.** *J. Chem. Soc.,* 1959, 2717
- **Mitsunobu, O.** *Synthesis,* 1981, 1
- **Szeja, W.** *Synthesis,* 1985, 983-985
- **Kwart, H. ; Hoffman, D. M.** *J. Org. Chem.,* 1966, vol. 31, 419-425
- **Gutshe, C. D.** *Organic Reactions,* 1954, vol. 8, 364
- **Rauter, A. P. ; Figueiredo, J. A. ; Ismael, M. I.** *Carbohydr. Res.,* 1989, vol. 188, 19-24
- **Dax, K. ; Rauter, A. P. ; Stütz, A. E. ; Weidmann, H.** *Liebigs Ann. Chem.,* 1981, 1768-1773
- **Robertson, J. L. ; Preisher, H.** Pesticide Bioassays with Arthropods. CRC Press, 1992
- **Finney, D. J.** Probit Analysis. Cambridge University Press, 1972, 79